# EUROPÄISCHE PATENTSCHRIFT

(11) **EP 1 388 325 B1**
(45) Veröffentlichungstag und Bekanntmachung des Hinweises auf die Patenterteilung: **22.03.2006**
(21) Anmeldenummer: 03017006.2
(22) Anmeldetag: 26.07.2003
(51) Int. Cl.: A61B 19/02

(54) **Folientasche zur Aufnahme von Verbandzeug**
Foil bag for dressing material
Sachet pour pansements

(30) Priorität: 09.08.2002 DE 20212269 U
(43) Veröffentlichungstag der Anmeldung: 11.02.2004
(73) Patentinhaber: Leina-Werke GmbH, 51570 Windeck-Rosbach (DE)
(72) Erfinder: Steinhauer, Hartmut, 57537 Forst (DE)
(74) Vertreter: Herden, Andreas F.

(56) Entgegenhaltungen:
- DE-U- 29 503 188
- US-A- 4 550 831
- US-A- 6 016 915

## Beschreibung

Die Erfindung betrifft eine Folientasche zur Aufnahme von Verbandzeug, insbesondere zur Einlage in einen Verbandkasten.

Folientaschen zur Aufnahme von Verbandzeug sind beispielsweise aus der DE 29503188 bekannt, welche eine Nothilfepackung mit einer zickzackförmig zusammenlegbaren flexiblen Materialbahn beschreibt.

In DE 29713404 ist eine aus mehreren Lagen gefaltete Nothilfetasche offenbart und auch in der DE 29606532 ist eine Nothilfetasche aus flexiblem Material beschrieben.

Die US 6016915 zeigt eine Nothilfepackung gemäß dem Oberbegriff des Anspruchs 1.

Nachteilig an den bekannten Folientaschen ist jedoch, dass die einzelnen Abschnitte der Tasche eine Einheit bilden. Bei Verbandkästen für Kraftfahrzeuge ist vorgeschrieben, die Sterilprodukte nach Ablauf eines Verfallsdatums auszutauschen. In Betracht kommt dann bei herkömmlichen Nothilfepackungen der Austausch der gesamten Folientasche. Nachteilig ist dann aber, dass auch Produkte die keinem Verfallsdatum unterliegen, mit ausgetauscht oder umständlich umgetütet werden müssen.

Aufgabe der Erfindung ist es, eine Folientasche zu schaffen, bei der einzelne Abschnitte leicht abgetrennt und ausgetauscht werden können. Zudem solle das in die Abschnitte einbrachte Verbandzeug auch leicht einzeln ausgetauscht und aufgefüllt werden können.

Diese Aufgabe wird gemäß der Erfindung mit den Merkmalen des Anspruches 1 gelöst.

Danach ist eine Folientasche zur Aufnahme von Verbandzeug aus einer flexiblen Materialbahn vorgesehen, die zumindest zwei voneinander trennbare Abschnitte aufweist, die ihrerseits zumindest eine Öffnung zur Einbringung des Verbandzeuges aufweisen.

Die Folientasche ist hierzu vorteilhaft in mindestens zwei trennbare Abschnitte unterteilt. Die Abschnitte können hierdudrch jeweils abgetrennt und ausgetauscht werden.
Die Abschnitte haben jeweils mindestens eine Öffnung, die einen Austausch einzelner Produkte ermöglicht, ohne die Folientasche zu beschädigen oder zu verändern.

Weiterbildungen und bevorzugte Ausführungsbeispiele der Erfindung sind den jeweiligen Unteransprüchen zu entnehmen.

Bei einer bevorzugten Ausführungsform der Erfindung sind die Abschnitte durch eine Perforationslinie voneinander trennbar. Eine solche Perforationslinie kann mit wenig Aufwand in eine Folienbahn gestanzt werden. An der Perforationslinie können die einzelnen Abschnitte leicht getrennt werden.

Bei einer Weiterbildung der Erfindung ist zwischen den Abschnitten zumindest ein Faltabschnitt, der keine Fächer aufweist, angeordnet. So kann die Folientasche leicht zusammengefaltet werden und zum Beispiel in einen Verbandkasten eingelegt werden. Beim Auseinanderfalten der Abschnitte ist das Verbandzeug geordnet und übersichtlich zugänglich.

Eine bevorzugte Ausführungsform der Folientasche besitzt als Öffnungen Schnittlinien auf einer Seite. Solche Schnittlinien können leicht auf einer Seite der Folienbahn angebracht werden. Bei der Herstellung kann zweckmäßigerweise eine Folie mit Schnittlinien mit einer anderen Folie ohne Schnittlinien verschweisst werden.

Bei einer zweckmäßigen Ausführungsform der Folientasche liegen die Öffnungen in etwa senkrecht zur jeweiligen Trennlinie. So sind die Öffnungen besonders gut zugänglich.

Bei einer weiteren bevorzugten Ausführungsform der Erfindung liegen die Öffnungen der Abschnitte in etwa auf der Mittellinie der Folientasche, so dass sich in jedem Abschnitt zwei etwa gleich große Fächer ergeben. Durch die etwa gleich großen Fächer ist das Verbandzeug gut geordnet.

Zweckmäßigerweise ist zumindest ein Abschnitt nur für Sterilprodukte vorgesehen. Dieser Abschnitt kann dann kurz vor oder nach Ablauf des Verfallsdatums abgetrennt und ausgetauscht werden.

Bei einer zweckmäßigen Ausführungsform ist die Folientasche einstückig. Eine solche Tasche ist besonders kostengünstig herstellbar, zum Beispiel durch das Aufeinanderbringen und Verschweissen von zwei Folien.

Bevorzugtes Material für die Folientasche ist Polyethylen (PE) oder Polyvinylchlorid (PVC).

Bei einer Weiterbildung der Erfindung weist die Folientasche zwei sich senkrecht überschneidende Perforationslinien auf und ist so in vier Abschnitte trennbar.

Die Erfindung wird nachfolgend anhand eines bevorzugten Ausführungsbeispiels anhand der beigefügten Zeichnung näher erläutert.

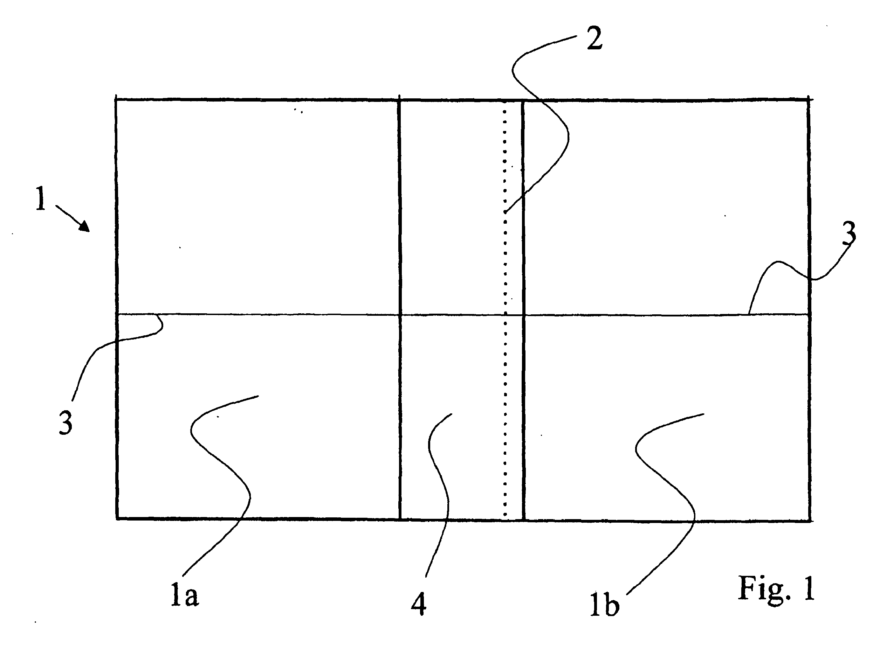

Fig. 1 zeigt eine erfindungsgemäße Folientasche in der Draufsicht.

Die Folientasche 1 ist in zwei Abschnitte 1a, 1b zur Aufnahme von Verbandzeug unterteilt. Die Abschnitte 1a, 1b weisen auf der Oberseite Schnittöffnungen 3 auf. Über diese Schnittöffnungen können einzelne Bestandteile des Verbandzeuges leicht ausgetauscht und nachgefüllt werden. Auf der Unterseite (nicht zu sehen) sind keine Schnittöffnungen vorgesehen.

Die Folientasche weist ferner einen Faltabschnitt 4 zum Zusammenfalten der beiden Abschnitte 1a, 1b auf. So kann die Folientasche auch in gefülltem Zustand leicht zusammengefaltet werden.

Der Faltabschnitt 4 weist eine Perforationslinie 2 auf. Entlang dieser Perforationslinie 2 können die Abschnitte 1a, 1b leicht getrennt werden. So können zum Beispiel in rechten Abschnitt 1b die Sterilprodukte eingebracht werden. Nach Ablauf des Verfallsdatums ist der Abschnitt abtrenn- und austauschbar.

## Patentansprüche

1. Folientasche (1) zur Aufnahme von Verbandzeug und zur Einlage in einen Verbandkasten ausgebildet, umfassend eine flexible Materialbahn,
wobei die Folientasche (1) zumindest zwei voneinander trennbare Abschnitte (1a, 1b) aufweist, wobei die Abschnitte (1a, 1b) durch eine Perforationslinie (2) voneinander trennbar sind, und wobei ein Abschnitt (1a, 1b) ausschließlich für Sterilprodukte vorgesehen ist und zwischen den Abschnitten (1a, 1b) zumindest ein Faltabschnitt (4) angeordnet ist, welcher keine Fächer aufweist, **dadurch gekennzeichnet, dass** die Abschnitte (1a, 1b) zumindest eine Öffnung (3) zur Einbringen des Verbandzeuges aufweisen.

2. Folientasche nach einem der vorhergehenden Ansprüche,
**dadurch gekennzeichnet,**
**dass** die Folientasche als Öffnungen Schnittlinien (3) auf einer Seite aufweist.

3. Folientasche nach einem der vorhergehenden Ansprüche,
**dadurch gekennzeichnet,**
**dass** die Öffnungen (3) in etwa senkrecht zur jeweiligen Trennlinie (2) liegen.

4. Folientasche nach einem der vorhergehenden Ansprüche,
**dadurch gekennzeichnet,**
**dass** die Öffnungen (3) der Abschnitte (1a, 1b) in etwa auf der Mittellinie der Folientasche liegen, so dass sich in jedem Abschnitt zwei etwa gleich große Fächer ergeben.

5. Folientasche nach einem der vorhergehenden Ansprüche,
**dadurch gekennzeichnet,**
**dass** die Folientasche einstückig ausgebildet ist.

6. Folientasche nach einem der vorhergehenden Ansprüche,
**dadurch gekennzeichnet,**
**dass** die Folientasche aus Polyethylen oder Polyvinylchlorid besteht.

7. Folientasche nach einem der vorhergehenden Ansprüche,
**dadurch gekennzeichnet,**
**dass** die Folientasche zwei sich senkrecht überschneidende Perforationslinien aufweist und in vier Abschnitte trennbar ist.

## Claims

1. Foil bag (1) for dressing material for arranging in a first aid box, comprising a flexible strip of material wherein the foil bag (1) comprises at least two sections (1a, 1b) adapted to be separated from each other, the sections (1a, 1b) are adapted to be separated from each other via a perforation line (2) and wherein one section (1a, 1b) is provided exclusively for sterile products and at least one folding section (4) is disposed between the sections (1a, 1b) wherein said folding section (4) does not have any compartments, **characterised in that** the sections (1a, 1b) have at least one opening (3) for inserting the dressing material.

2. Foil bag according to one of the preceding claims,
**characterised in that**
the foil bag has cut lines (3) on one side as openings.

3. Foil bag according to one of the preceding claims,
**characterised in that**
the openings (3) lie approximately perpendicular to the respective separating line (2).

4. Foil bag according to one of the preceding claims,
**characterised in that**
the openings (3) of the sections (1a, 1b) lie approximately on the centre line of the foil bag in such a way that two compartments of approximately the same size result in each section.

5. Foil bag according to one of the preceding claims,
**characterised in that**
the foil bag is formed in one piece.

6. Foil bag according to one of the preceding claims,
**characterised in that**
the foil bag is made of polyethylene or polyvinyl chloride.

7. Foil bag according to one of the preceding claims,
**characterised in that**
the foil bag has two perpendicularly overlapping perforation lines and is adapted to be separated into four sections.

## Revendications

1. Sachet (1) destiné à recevoir un pansement et conçu pour être inséré dans une boîte à pansements, comportant une bande de matériau flexible,
le sachet (1) comprenant au moins deux sections (1a, 1b) pouvant être séparées l'une de l'autre, les sections (1a, 1b) pouvant être séparées l'une de l'autre par une ligne de perforation (2), et une section (1a, 1b) étant prévue exclusivement pour des produits stériles et au moins une section de pliage (4) étant disposée entre les sections (1a, 1b), laquelle ne comprend aucun compartiment, **caractérisé en ce que** les sections (1a, 1b) comprennent au moins une ouverture (3) destinée à y introduire le pansement.

2. Sachet selon l'une des revendications précédentes, **caractérisé en ce que** le sachet comprend comme ouvertures des lignes de coupe (3) sur une face.

3. Sachet selon l'une des revendications précédentes, **caractérisé en ce que** les ouvertures (3) se situent approximativement perpendiculairement à la ligne de séparation (2) respective.

4. Sachet selon l'une des revendications précédentes, **caractérisé en ce que** les ouvertures (3) des sections (1a, 1b) se situent approximativement sur la ligne médiane du sachet, de sorte que cela engendre dans chaque section deux compartiments présentant approximativement la même dimension.

5. Sachet selon l'une des revendications précédentes, **caractérisé en ce que** le sachet est conçu en une seule pièce.

6. Sachet selon l'une des revendications précédentes, **caractérisé en ce que** le sachet est constitué de polyéthylène ou de chlorure de polyvinyle.

7. Sachet selon l'une des revendications précédentes, **caractérisé en ce que** le sachet comprend deux lignes de perforation se croisant verticalement et peut être séparé en quatre sections.
